# EUROPÄISCHE PATENTSCHRIFT

(11) **EP 0 920 426 B1**
(45) Veröffentlichungstag und Bekanntmachung des Hinweises auf die Patenterteilung: **01.10.2003**
(21) Anmeldenummer: 97940080.1
(22) Anmeldetag: 13.08.1997
(51) Int. Cl.: C07D 401/14, A61K 31/425

(54) **NEUE THIAZOL-DERIVATE MIT PHOSPHODIESTERASE INHIBIERENDER WIRKUNG**
NEW THIAZOLE DERIVATIVES WITH PHOSPHODIESTERASE-INHIBITING EFFECT
NOUVEAUX DERIVES DE THIAZOL A EFFET INHIBANT LA PHOSPHODIESTERASE

(30) Priorität: 26.08.1996 DE 19634411; 28.08.1996 EP 96113737
(43) Veröffentlichungstag der Anmeldung: 09.06.1999
(73) Patentinhaber: ALTANA Pharma AG, 78467 Konstanz (DE)
(72) Erfinder: AMSCHLER, Hermann, deceased (DE); MARTIN, Thomas, D-78462 Konstanz (DE); FLOCKERZI, Dieter, D-78476 Allensbach (DE); Schmidt, Beate, D-78476 Allensbach (DE); THIBAUT, Ulrich, D-78464 Konstanz (DE); HATZELMANN, Armin, D-78467 Konstanz (DE); BOSS, Hildegard, D-78476 Allensbach (DE); HÄFNER, Dietrich, D-78464 Konstanz (DE); KLEY, Hans-Peter, D-78476 Allensbach (DE); BEUME, Rolf, D-78465 Konstanz (DE); BÄR, Thomas, D-78479 Reichenau (DE); ULRICH, Wolf-Rüdiger, D-78464 Konstanz (DE)
(86) Internationale Anmeldenummer: EP9704435
(87) Internationale Veröffentlichungsnummer: WO98008841

(56) Entgegenhaltungen:
- EP-A- 0 513 387
- EP-A- 0 600 092
- EP-A- 0 636 626
- WO-A-96/03392
- CHIHIRO,M. ET AL.: "Novel Thiazole Derivatives as Inhibitors of Superoxide Production by Human Neutrophils: Synthesis and Structure-Activity Relationships" J.MED.CHEM., Bd. 38, 1995, WASHINGTON, Seiten 353-358, XP002024855 in der Anmeldung erwähnt
- DUMAITRE,B. ET AL.: "Synthesis and Cyclic GMP Phosphodiesterase Inhibitory Activity of a Series of 6-Phenylpyrazolo[3,4-d] pyrimidones" J.MED.CHEM., Bd. 39, Nr. 8, 1996, WASHINGTON, Seiten 1635-1644, XP002024856
- PALFREYMAN,M.N. ET AL.: "Phosphodiesterase Type IV Inhibitors" PROG.MED.CHEM., Bd. 33, 1996, AMSTERDAM, Seiten 1-52, XP000650817

## Beschreibung

### Anwendungsgebiet der Erfindung

Die Erfindung betrifft neue Thiazol-Derivate, die in der pharmazeutischen Industrie zur Herstellung von Medikamenten verwendet werden.

### Bekannter technischer Hintergrund

In der japanischen Patentschrift JP 46-15935 werden substituierte 4-(Carboxyphenyl)thiazole und ihre Verwendung zur Behandlung von Thrombose, Arteriosklerose, Magengeschwüren und Hypersekretion beschrieben. In den europäischen Patentanmeldungen EP 0 513387. EP 0 600 092 und in J. Med. Chem. 38, 353-358 (1995) werden unter anderem 2-(substituiertes Phenyl)thiazolderivate, 2-(substituiertes 2,3-Dihydrobenzofuran)thiazolderivate und ihre Verwendung als Inhibitoren der Sauerstoffradikalfreisetzung durch Neutrophile beschrieben. Die Verbindungen werden daher als geeignet zur Behandlung akut entzündlicher Prozesse wie Ischämien und Reperfusionsschäden beschrieben. In der internationalen Patentanmeldung WO 96/03392 werden mehrfach substituierte Thiazolderivate als Entzündungshemmer beschrieben. In der europäischen Patentanmeldung EP 0 636 626 und in J. Med. Chem. 39, 1635-1644 (1996) werden Pyrazolo[3,4-d]pyrimidin-4-on Derivate, die unter anderem durch einen Thiazolrest substituiert sein können, als selektive Inhibitoren der cGMP spezifischen Phosphodiesterase beschrieben. Aus Progr. Med. Chem. 33, 1-52 (1996) ist bekannt, dass Phosphodiesterase-lnhibitoren eine anti-inflammatorische Wirkung besitzen und auch die Superoxyradikalanion-Produktion unterdrücken.

### Beschreibung der Erfindung

Es wurde nun überraschenderweise gefunden, daß die neuen, nachfolgend näher beschriebenen Thiazol-Derivate, die sich von den vorveröffentlichten Thiazolen insbesondere durch die Substituenten am 2-(2,3-Dihydrobenzofuranring) unterscheiden, selektive Inhibitoren der Phosphodiesterase IV sind.

Gegenstand der Erfindung sind somit Verbindungen der Formel l (siehe beigefügtes Formelblatt), worin
- R1: 1-4C-Alkoxy bedeutet,
- R2 und R3: gemeinsam und unter Einschluß der beiden Kohlenstoffatome, an die sie gebunden sind; einen Cyclopentanring darstellen,
- R4: einen durch R41 substituierten Phenylring bedeutet oder durch R44 substituiertes Pyridin darstellt, wobei
- R41: Wasserstoff, Carboxyl, 1-4C-Alkoxycarbonyl, Carbamoyl, Hydroxysulfonyl, Sulfamoyl oder Hydroxy, und
- R44: Wasserstoff, Carboxyl, 1-4C-Alkoxycarbonyl, Carbamoyl, Hydroxysulfonyl, Sulfamoyl oder Hydroxy bedeutet,
- R5: Wasserstoff bedeutet,
- n: 0 bedeutet,
sowie die Salze dieser Verbindungen.

1 -4C-Alkoxy steht für einen Rest, der neben dem Sauerstoffatom einen geradkettigen oder verzweigten Alkylrest mit 1 bis 4 Kohlenstoffatomen enthält. Als Alkylreste mit 1 bis 4 Kohlenstoffatomen seien hierbei beispielsweise genannt der Butyl-, iso-Butyl-, sec.-Butyl-, tert-Bulyl-, Propyl-, Isopropyl-, Ethyl- und der Methylrest.

1-4C-Alkyl steht für geradkettige oder verzweigte Alkylreste mit 1 bis 4 Kohlenstoffatomen. Beispielsweise seien genannt der Butyl-, der iso-Butyl-, der sec.-Butyl-, der tert.-Butyl-, der Propyl-, der iso-Propyl-, der Ethyl- und insbesondere der Methylrest.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-CO-) und der Ethoxycarbonylrest (CH₃CH₂O-CO-) genannt.

Die Anknüpfung des Substituenten R4 an den Rest der Verbindungen der Formel I kann über jede geeignete Ringposition des Phenylrings bzw. des Heterocyclus erfolgen.

Beispielhaft für R4 seien die Reste Phenyl, 3-Acetoxyphenyl, 3-Acetylphenyl, 2-Carboxyphenyl, 3-Carboxyphenyl, 4-Carboxyphenyl, 2-Hydroxyphenyl, 3-Hydroxyphenyl, 4-Hydroxyphenyl, 2-Ethoxycarbonylphenyl, 3-Ethoxycarbonylphenyl, 4-Ethoxycarbonylphenyl, 2-Carbamoylphenyl, 3-Carbamoylphenyl, 4-Carbamoylphenyl, 2-Sulfamoylphenyl, 3-Sulfamoylphenyl, 4-Sulfamoylphenyl, 3-Methoxycarbonylpyridin-5-yl, 2-Methoxycarbonylpyridin-4-yl, 2-Methoxycarbonylpyridin-5-yl, 6-Carboxypyridin-2-yl, 6-Ethoxycarbonylpyridin-2-yl, 3-Carboxypyridin-5-yl, 4-Carboxypyridin-2-yl, 5-Carboxypyridin-2-yl, 3-Carboxypyridin-2-yl, 2-Carboxypyridin-4-yl, 2-Carboxypyridin-5-yl, 3-Acetylpyrid-2-yl, 5-Hydroxypyridin-2-yl, 2-Pyridyl, 3-Pyridyl, 4-Pyridyl, 2-Acetoxypyridin-4-yl und 3-Methoxycarbonylpyridin-2-yl genannt.

Als Salze kommen für Verbindungen der Formel l - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure. Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Hervorzuhebende Verbindunaen der Formel l sind solche, worin
- R1: Methoxy,
- R2 und R3: gemeinsam und unter Einschluß der beiden Kohlenstoffatome, an die sie gebunden sind, einen Cyclopentanring darstellen,
- R4: einen durch R41 substituierten Phenylring bedeutet oder durch R44 substituiertes Pyridin darstellt, wobei
- R41: Carboxyl oder Carbamoyl und
- R44: Wasserstoff, Carboxyl oder 1-4C-Alkoxycarbonyl bedeuten,
- R5: Wasserstoff bedeutet,
- n: 0 bedeutet,
sowie die Salze dieser Verbindungen.

Beispielhafte erfindungsmäßige Verbindungen sind in den folgenden Tabellen aufgeführt:

**Tabelle 1**

| Verbindungen der Formel l mit R4 = 3-Pyridyl, 4-Pyridyl, 5-Carboxypyrid-3-yl, 5-Carbamoylpyrid-3-yl oder 5-Methoxycarbonylpyrid-3-yl, R5 = H, n = 0 und den folgenden weiteren Substituentenbedeutungen: | | |
|---|---|---|
| **R1** | **R2** | **R3** |
| OCH₃ | CH₂CH₂CH₂ | |
| OC₂H₅ | CH₂CH₂CH₂ | |
| OCH₂C₃H₅ | CH₂CH₂CH₂ | |

**Tabelle 2**

| Verbindungen der Formel l mit R4 = Phenyl, 3-Carboxyphenyl, 4-Carboxyphenyl, 3-Carbamoylphenyl oder 4-Carbamoylphenyl, R5 = H, n = 0 und den folgenden weiteren Substituentenbedeutungen: | | |
|---|---|---|
| **R1** | **R2** | **R3** |
| OCH₃ | CH₂CH₂CH₂ | |
| OC₂H₅ | CH₂CH₂CH₂ | |
| OCH₂C₃H₅ | CH₂CH₂CH₂ | |

Bei den Verbindungen der Formel I handelt es sich - sofern die Substitutionen -R2 und -CH₂R3 nicht identisch sind - um chirale Verbindungen. Die Erfindung umfaßt daher sowohl die reinen Enantiomeren als auch deren Gemische in jedem Mischungsverhältnis, einschließlich der Racemate. Die Enantiomeren können in an sich bekannter Weise (beispielsweise durch Herstellung und Trennung entsprechender diastereoisomerer Verbindungen) separiert werden.

Weiterer Gegenstand der Erfindung ist ein Verfahren zur Herstellung der Verbindungen der Formel 1 und ihrer Salze. Das Verfahren ist dadurch gekennzeichnet, daß man Verbindungen der Formel II (siehe beigefügtes Formelblatt), in denen R1, R2 und R3 die oben angegebenen Bedeutungen haben und Z die Gruppe -C(S)-NH₂ bedeutet mit Verbindungen der Formel III (siehe beigefügtes Formelblatt), in denen R4, R5 und n die oben angegebenen Bedeutungen haben und Y eine geeignete Abgangsgruppe darstellt, umsetzt, und daß man gewünschtenfalls anschließend erhaltene Verbindungen der Formel l in ihre Salze oder daß man gewünschtenfalls anschließend erhaltene Salze der Verbindungen der Formel I in die freien Verbindungen überführt.

Welche Abgangsgruppen Y geeignet sind, ist dem Fachmann aufgrund seines Fachwissens geläufig. Beispielsweise wird von geeigneten Verbindungen der Formel lll ausgegangen, in denen Y die Bedeutung Halogen insbesondere Brom oder Chlor hat. Im übrigen erfolgt die Umsetzung auf eine dem Fachmann an sich vertraute Weise (z.B. wie in EP 0 513 387 und EP 0 600 092 beschrieben) in einem geeigneten Lösungsmittel und in Gegenwart oder Abwesenheit einer Base, vorzugsweise bei Reaktionstemperaturen zwischen Raumtemperatur und der Siedetemperatur des verwendeten Lösungsmittels und bei Reaktionszeiten zwischen einer Stunde und zwei Tagen. Geeignete Lösungsmittel sind beispielsweise Alkohole wie Methanol, Ethanol oder Propanol, cyclische Kohlenwasserstoffe wie Toluol oder Xylol, Ether wie Diethylether, Tetrahydrofuran oder Dioxan, halogenierte Kohlenwasserstoffe wie Dichlormethan oder Chloroform, polare Lösungsmittel wie Dimethylformamid, Acetonitril oder Dimethylsulfoxid oder gewünschtenfalls auch Gemische der genannten Lösungsmittel. Bevorzugte Basen die Verwendung finden sind Stickstoffbasen wie Triethylamin, Ethyldiisopropylamin, N-Methylmorpholin oder Pyridin. Die Basen können dabei im äquimolaren Verhältnis (bezogen auf Verbindungen der Formel III) oder vorzugsweise im Überschuß zugesetzt werden.

Gewünschtenfalls können erhaltene Verbindungen der Formel l auch durch Anwendung dem Fachmann bekannter Methoden in andere Verbindungen der Formel l übergeführt werden. Beispielhaft sei die Herstellung von Carbonsäureamiden der Formel l aus den entsprechenden Carbonsäuren der Formel l genannt. Dazu können die Carbonsäuren der Formel I mit geeigneten Aminen in einer Weise, wie sie dem Fachmann zur Synthese von Carbonsäureamiden bekannt ist, umgesetzt werden. Gewünschtenfalls wird die Carbonsäure der Formel 1 vor der Aminolyse in ein geeignet aktiviertes Derivat, beispielsweise ein entsprechendes Säurehalogenid übergeführt. Als geeignete Amine die eingesetzt werden können seien beispielsweise Ammoniak, Methylamin oder Ethylamin genannt.

Beispielhaft sei auch die Herstellung von Carbonsäuren der Formel l aus entsprechenden Estern der Formel 1 erwähnt, beispielsweise durch Verseifung auf eine dem Fachmann bekannte Weise, beispielsweise so wie in den Beispielen beschrieben.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die Verbindungen der Formel ll, worin Z die Gruppe -C(S)-NH₂ bedeutet, können auf dem Fachmann bekannte Weise, zum Beispiel durch Addition von Schwefelwasserstoff an Verbindungen der Formel II, worin Z eine Nitrilgruppe (-CN) darstellt, hergestellt werden [z.B. wie beschrieben in: W. Christ, D. Rakow, S. Strauss, J. Heterocycl. Chem. 11, 397 (1974)].

Die Verbindungen der Formel II, worin Z eine Nitrilgruppe bedeutet, können wie in der Literatur beschrieben (T. Savaie, T.lshiguro, K. Kawashima, K. Morita; Tetrahedron Lett. 1973, 2121-2124) aus den entsprechenden Verbindungen der Formel II, in denen Z die Bedeutung Carbamoyl [-C(0)-NH₂] hat, hergestellt werden.

Die Verbindungen der Formel II, worin Z die Bedeutung Carbamoyl hat, lassen sich aus den Verbindungen der Formel II, in denen Z die Bedeutung Carboxyl hat, in einer dem Fachmann vertrauten Weise, beispielsweise so wie in den nachfolgenden Beispielen beschrieben, darstellen.

Verbindungen der Formel II, worin Z Carboxyl bedeutet, sind entweder bekannt aus der internationalen Patentanmeldung WO96/03399 oder können auf analoge Weise hergestellt werden.

Die Verbindungen der Formel III, in denen R4 und R5 die oben angegebenen Bedeutungen haben, n = 0 ist und Y Halogen, insbesondere Chlor oder Brom bedeutet, sind entweder bekannt (z.B. aus EP 0 513 387 und EP 0 600 092) oder können auf bekannte Weise erhalten werden, beispielsweise durch Chlorierung respektive Bromierung entsprechender Verbindungen der Formel lll, worin Y die Bedeutung Wasserstoff hat.

Alternativ können Verbindungen der Formel III, in denen R4 und n die oben angegebenen Bedeutungen haben, R5 Wasserstoff und Y insbesondere Chlor oder Brom bedeutet auch durch Umsetzung von Verbindungen der Formel llla (siehe beigefügtes Formelblatt), in denen R4 und n die oben angegebenen Bedeutungen haben und A eine geeignete Abgangsgruppe, insbesondere Chlor oder Brom bedeutet. mit Diazomethan und anschließender Behandlung mit HCI beziehungsweise HBr erhalten werden.

Die folgenden Beispiele erläutern die Erfindung näher, ohne sie einzuschränken. Ebenso können weitere Verbindungen der Formel I, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, h für Stunde(n), RT für Raumtemperatur, Min. für Minute(n), Tol. für Toluol, EA für Ethylacetat und PE für Petrolether. Die in den Beispielen genannten Verbindungen und ihre Salze sind bevorzugter Gegenstand der Erfindung.

### Beispiele

### Endprodukte

### 1. 3-[2-(2,3-Dihydro-7-methoxy-benzofuran-2-spiro-1'-cyclopentan-4-yl)thiazol-4-yl]-pyridin

260 mg (1,0 mmol) 2,3-Dihydro-7-methoxy-benzofuran-2-spiro-1'-cyclopentan-4-yl-thiocarbonsäureamid und 340 mg (1,2 mmol) 2-Brom-1-(pyridin-3-yl)ethanon Hydrobromid werden in 30 ml Ethanol 4 h bei RT gerührt. Man engt im Vakuum ein, suspendiert in H₂O, stellt alkalisch und extrahiert mit Ethylacetat. Die organische Phase wird über MgSO₄ getrocknet, eingedampft und der feste Rückstand aus 10 ml Ethanol umkristallisiert. Man erhält 176 mg (48 %) der Titelverbindung vom Schmp. 160-162°C.

### 2. 4-[2-(2,3-Dihydro-7-methoxy-benzofuran-2-spiro-1-cyclopentan-4-yl)thiazol-4-yl]-pyridin Hydrobromid

500 mg (1,9 mmol) 2,3-Dihydro-7-methoxy-benzofuran-2-spiro-1'-cyclopentan-4-yl-thiocarbonsäureamid und 533 mg (1,9 mmol) 2-Brom-1-(pyridin-4-yl)ethanon Hydrobromid werden in 20 ml Ethanol 4 h bei 40°C gerührt. Man saugt ab, wäscht den Niederschlag mit Ethanol und erhält 630 mg (91 %) der Titelverbindung vom Schmp. 205-207°C.

### 3. 3-[2-(2,3-Dihydro-7-methoxy-benzofuran-2-spiro-1'-cyclopentan-4-yl)thiazol-4-yl]-pyridin-5-carbonsäuremethylester Hydrobromid

1,47 g (5,59 mmol) 2,3-Dihydro-7-methoxy-benzofuran-2-spiro-1'-cyclopentan-4-yl-thiocarbonsäureamid und 1,45 g (5,59 mmol) 5-Bromacetyl-nicotinsäuremethylester werden in 30 ml Ethanol 1,5 h bei 70°C gerührt. Der entstandene Niederschlag wird abgesaugt, in Diisopropylether umkristallisiert und man erhält 1,38 g (48 %) der Titelverbindung vom Schmp. 211°C.

### 4. 3-[2-(2,3-Dihydro-7-methoxy-benzofuran-2-spiro-1'-cyclopentan-4-yl)thiazol-4-yl]-pyridin-5-carbonsäure

1,20 g (2,84 mmol) der Verbindung 3 und 312 mg (13,05 mmol) Lithiumhydroxyd werden in einem Gemisch aus Methanol und Wasser suspendiert. Nach 3 h Rühren bei 40°C wird die inzwischen klare Reaktionslösung mit 2 N HCl neutralisiert und eingeengt. Man verteilt zwischen Ethylacetat und Wasser. Die organische Phase enthält sowohl gelöstes als auch ungelöstes Produkt. Der Feststoff wird abfiltriert, die Lösung über MgSO₄ getrocknet und eingeengt. Man verrührt das vereinigte Rohprodukt in Ethanol und erhält 1,03 g (89 %) der Titelverbindung vom Schmp. > 270°C.

### 5. 3-[2-(2,3-Dihydro-7-methoxy-benzofuran-2-spiro-1'-cyclopentan-4-yl)thiazol-4-yl]-benzoesäure

260 mg (1,0 mmol) 2,3-Dihydro-7-methoxy-benzofuran-2-spiro-1'-cyclopentan-4-yl-thiocarbonsäureamid und 360 mg 3-Bromacetyl-benzoesäure werden unter Zusatz von 0,5 ml Triethylamin in 20 ml Ethanol 2 h bei RT gerührt. Man engt ein, suspendiert in H₂O und säuert mit konz. HCI an. Nach mehrmaliger Extraktion mit Ethylacetat werden die vereinigten organischen Phasen über MgSO₄ getrocknet und eingeengt. Der Rückstand wird mit 15 ml Ethanol ausgekocht und man erhält 180 mg (44 %) der Titelverbindung vom Schmp. 256-259°C.

### 6. 3-[2-(2,3-Dihydro-7-methoxy-benzofuran-2-spiro-1'-cyclopentan-4-yl)thiazol-4-yl]-benzamid

800 mg (1,96 mmol) der Verbindung **5** werden in 10 ml Thionylchlorid 1 h zum Rückfluß erhitzt. Überschüssiges Thionylchlorid wird im Vakuum abdestilliert und der Rückstand in 30 ml eisgekühltem konzentriertem Ammoniak 1 h lang geschüttelt. Man extrahiert mit Dichlormethan und chromatographiert über Kieselgel (Ethylacetat). Nach zweimaligem Umkristallisieren aus i-Butylmethylketon erhält man 60 mg (8 %) der Titelverbindung vom Schmp. 246°C.

### 7. 4-[2-(2,3-Dihydro-7-methoxy-benzofuran-2-spiro-1'-cyclopentan-4-yl)-thiazol-4-yl]-benzoesäure

2,0 g (7,59 mmol) 2,3-Dihydro-7-methoxy-benzofuran-2-spiro-1'-cyclopentan-4-yl-thiocarbonsäureamid und 1,84 g 4-Bromacetyl-benzoesäure werden unter Zusatz von 768 mg (7,59 mmol) N-Methylmorpholin in 50 ml Ethanol 40 Min. bei 70°C gerührt. Nach Abkühlen der Reaktionslösung wird der Niederschlag abgesaugt, mit Ethanol gewaschen und mit Diethylether verrührt. Man erhält 180 mg (44 %) der Titelverbindung vom Schmp. > 250°C.

### 8. 4-[2-(2,3-Dihydro-7-methoxy-benzofuran-2-spiro-1'-cyclopentan-4-yl)thiazol-4-yl]-benzamid

600 mg (1,47 mmol) der Verbindung 7 werden in 3 ml Thionylchlorid 1 h zum Rückfluß erhitzt. Überschüssiges Thionylchlorid wird im Vakuum abdestilliert und der Rückstand in 10 ml Aceton aufgenommen. Unter Eiskühlung werden 10 ml konz. Ammoniak zugetropft und 20 Min. im Eisbad nachgerührt. Der Niederschlag wird abgesaugt und über Kieselgel chromatographiert (Tol:EA:NEt₃ = 70:29:1). Man erhält 100 mg (17 %) der Titelverbindung vom Schmp. 245°C.

### Ausgangsverbindungen

### A. 2,3-Dihydro-7-methoxy-benzofuran-2-spiro-1'-Cyclopentan-4-yl-carbonsäurethioamid

960 mg (4,2 mmol) 4-Cyano-2,3-dihydro-7-methoxy-benzofuran-2-spiro-1'-cyclopentan werden in 10 ml Triethylamin und 10 ml Pyridin gelöst. Man sättigt mit H₂S und rührt 4 Tage bei RT. Das Reaktionsgemisch wird in Ethylacetat aufgenommen und mit verd. HCI vom Pyridin befreit. Nach Einengen und Kristallisation aus PE:Tol = 1:1 erhält man 860 mg (78 %) der Titelverbindung vom Schmp. 187-190°C.

### B. 4-Cyano-2,3-dihydro-7-methoxy-benzofuran-2-spiro-1'-cyclopentan

1,4g (6,0 mmol) 2,3-Dihydro-7-methoxy-benzofuran-2-spiro-1'-cyclopentan-carbonsäureamid werden in 25 ml CHCl₃ gelöst und mit 210 mg (1,0 mmol) Benzyltriethylammoniumchlorid versetzt. Unter Eiskühlung werden 5 ml 50%ige NaOH zugegeben und 3 h bei 15°C gerührt. Man versetzt mit H₂O, extrahiert mit Ethylacetat und chromatographiert über Kieselgel (Tol.). Die Kristallisation erfolgt aus 5 ml Petroläther und man erhält 998 mg (77 %) der Titelverbindung vom Schmp. 74-76°C.

### C. 2,3-Dihydro-7-methoxy-benzofuran-3-spiro-1'-cyclopentan-4-yl-carbonsäureamid

3,5 g (14,0 mmol) 2,3-Dihydro-7-methoxy-benzofuran-2-spiro-1'-cyclopentan-4-yl-carbonsäure werden mit 10 ml (ca. 140 mmol) SOCl₂ behandelt. Das überschüssige SOCl₂ wird im Vakuum abdestilliert und der Rückstand in 20 ml Aceton aufgenommen. Man versetzt unter Eiskühlung mit 10 ml konz. NH₃ und läßt 1 h nachrühren. Das Aceton wird abdestilliert und der Rückstand zwischen Ethylacetat und 0,5 N NaOH verteilt. Die getrocknete organische Phase wird aus 5 ml 50%igem Methanol kristallisiert und man erhält 115 mg (48 %) der Titelverbindung vom Schmp. 149-151°C.

### D. 2,3-Dihydro-7-methoxy-benzofuran-2-spiro-1'-cyclopentan-4-yl-carbonsäure

Die Darstellung der Titelverbindung ist in der internationalen Patentanmeldung WO96/03399 beschrieben.

### E. 2-Brom-1-(pyridin-3-yl)-ethanon Hydrobromid

Die Darstellung der Titelverbindung ist literaturbekannt. Lit.: A. Dornow et. al., Chem. Ber. 84, 148 (1951).

### F. 2-Brom-1-(pyridin-4-yl)-ethanon Hydrobromid

Die Darstellung der Titelverbindung ist literaturbekannt. Lit.: G. Sarodnick, G. Kempter; Pharmazie 40, 384-387 (1985).

### G. 5-Bromacetyl-nicotinsäuremethylester

Die Darstellung der Titelverbindung ist literaturbekannt. Lit.: S. McLean; Can. J. Chem. 54, 1262-1277 (1976).

### H. 3-Bromacetyl-benzoesäure

Die Darstellung der Titelverbindung ist literaturbekannt. Lit.: Schmied, Gröding, Monatshefte Chem. 84, 491, 496 (1953).

### I. 4-Bromacetyl-benzoesäure

Die Darstellung der Titelverbindung ist literaturbekannt. Lit.: C. Masatoshi et. al.; J. Med. Chem. 38, 353-358 (1995).

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs IV) eignen sie sich einerseits als Bronchialtherapeutika (zur Behandlung von A-temwegsobstruktionen aufgrund ihrer dilatierenden aber auch aufgrund ihrer-atemfrequenz- bzw. atemantriebssteigemden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine, Chemokine, alpha-, beta- und gamma-lnterfero Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen; oder auch Erkrankungen des ZNS, wie beispielsweise Depressionen oder arteriosklerotische Demenz.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die HerStellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 1 mg pro Sprühstoß. Die übliche Dosis bei systemischer Therapie p.o. oder i.v. liegt zwischen 0,1 und 200 mg pro Applikation.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" 57: 47-76, 1992; ed. Coffey RG (Marcel Decker, Inc., New York-Basel-Hong Kong)].

Substanzen, welche die Chemolumineszenz sowie die Zytokinsekretion und die Sekretion entzündungssteigemder Mediatoren an Entzündungszellen, insbesonders neutrophilen und eosinophilen Granulozyten hemmen, sind solche, welche die PDE IV hemmen. Dieses lsoenzym der Phosphodiesterase-Famillen ist besonders in Granulozyten vertreten. Dessen Hemmung führt zur Erhöhung der intrazellulären zyklischen AMP-Konzentration und damit zur Hemmung der zellulären Aktivierung. Die PDE lV-Hemmung durch die erfindungsgemäßen Substanzen ist damit ein zentraler Indikator für die Unterdrückung von entzündlichen Prozessen. (**Giembycz MA**, Could isoenzyme-selective phosphodiesterase inhibitors render bronchodilatory therapy redundant in the treatment of bronchial asthma?. Biochem Pharmacol 43: 2041-2051, 1992; **Torphy TJ** et al., Phosphodiesterase inhibitors: new opportunities for treatment of asthma. Thorax 46: 512-523, 1991; **Schudt C** et al., Zardaverine: a cyclic AMP PDE III/IV inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Caᵢ. Naunyn-Schmiedebergs Arch Pharmacol 344: 682-690, 1991; **Nielson CP** et al., Effects of selective phosphodiesterase inhibitors on polymorphonuclear leukocyte respiratory burst. J Allergy Clin Immunol 86: 801-808, 1990; **Schade** et al., The specific type III and IV phosphodiesterase inhibitor zardaverine suppress formation of tumor necrosis factor by macrophages. European Journal of Pharmacology 230: 9-14, 1993).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. 311, 193-198, 1980). Hierbei erfolgt im ersten Schritt die PDE-Reaktion. In einem zweiten Schritt wird das entstandene 5'-Nukleotid durch eine 5'-Nukleotidase des Schlangengiftes von ophiophagus hannah (King Cobra) zum ungeladenen Nukleosid gespalten. Im dritten Schritt wird das Nukleosid auf lonenaustauschsäulen vom verbliebenen geladenen Substrat getrennt. Die Säulen werden mit 2 ml 30 mM Ammonium formiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle 1, in der die Nummern der Verbindungen den Nummern der Beispiele entsprechen.

**Tabelle 1**

| **Hemmung der PDE lV-Aktivität** | |
|---|---|
| **Verbindung** | **-log IC**_{**50**} |
| 1 | 7.75 |
| 2 | 7.09 |

## Patentansprüche

1. Verbindungen der Formel l. 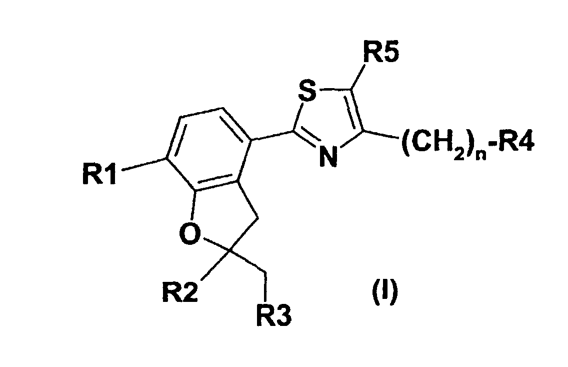 worin
R1 1-4C-Alkoxy bedeutet,
R2 und R3 gemeinsam und unter Einschluß der beiden Kohlenstoffatome, an die sie gebunden sind, einen Cyclopentanring darstellen,
R4 einen durch R41 substituierten Phenylring bedeutet oder durch R44 substituiertes Pyridin darstellt, wobei
R41 Wasserstoff, Carboxyl, 1-4C-Alkoxycarbonyl, Carbamoyl, Hydroxysulfonyl, Sulfamoyl oder Hydroxy, und
R44 Wasserstoff, Carboxyl, 1-4C-Alkoxycarbonyl, Carbamoyl, Hydroxysulfonyl, Sulfamoyl oder Hydroxy bedeutet,
R5 Wasserstoff bedeutet,
n 0 bedeutet,
sowie die Salze dieser Verbindungen.

2. Verbindungen der Formel I nach Anspruch 1, in denen
R1 Methoxy,
R2 und R3 gemeinsam und unter Einschluß der beiden Kohlenstoffatome, an die sie gebunden sind, einen Cyclopentanring darstellen,
R4 einen durch R41 substituierten Phenylring bedeutet oder durch R44 substituiertes Pyridin darstellt, wobei
R41 Carboxyl oder Carbamoyl und .
R44 Wasserstoff, Carboxyl oder 1-4C-Alkoxycarbonyl bedeuten,
R5 Wasserstoff bedeutet,
n 0 bedeutet,
sowie die Salze dieser Verbindungen.

3. Arzneimittel enthaltend eine oder mehrere Verbindungen nach Anspruch 1, zusammen mit üblichen pharmazeutischen Hilfs- und/oder Trägerstoffen.

4. Verbindungen nach Anspruch 1 zur Anwendung bei der Behandlung von Krankheiten.

5. Verwendung von Verbindungen nach Anspruch 1 zur Herstellung von Arzneimitteln für die Behandlung von Atemwegserkrankungen.

## Claims

1. Compounds of the formula I 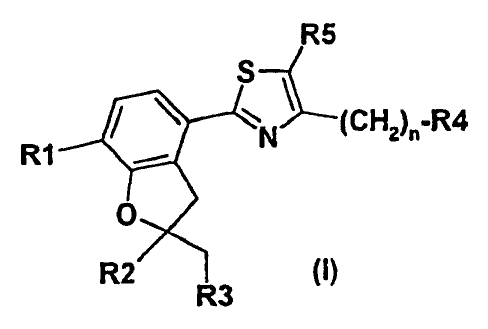 in which
R1 is 1-4C-alkoxy,
R2 and R3, together and including the two carbon atoms to which they are bonded, are a cyclopentane ring,
R4 is a phenyl ring substituted by R41 or pyridine substituted by R44, where
R41 is hydrogen, carboxyl, 1-4C-alkoxycarbonyl, carbamoyl, hydroxysulfonyl, sulfamoyl or hydroxyl and
R44 is hydrogen, carboxyl, 1-4C-alkoxycarbonyl, carbamoyl, hydroxysulfonyl, sulfamoyl or hydroxyl,
R5 is hydrogen,
n is 0,
and the salts of these compounds.

2. Compounds of the formula I as claimed in claim 1, in which
R1 is methoxy,
R2 and R3, together and including the two carbon atoms to which they are bonded, are a cyclopentane ring,
R4 is a phenyl ring substituted by R41 or pyridine substituted by R44, where
R41 is carboxyl or carbamoyl and
R44 is hydrogen, carboxyl or 1-4C-alkoxycarbonyl,
R5 is hydrogen,
n is 0,
and the salts of these compounds.

3. A medicament comprising one or more compounds as claimed in claim 1, together with customary pharmaceutical auxiliaries and/or excipients.

4. A compound as claimed in claim 1 for use in the treatment of illnesses.

5. The use of compounds as claimed in claim 1 for the production of medicaments for the treatment of airway disorders.

## Revendications

1. Composés de formule I 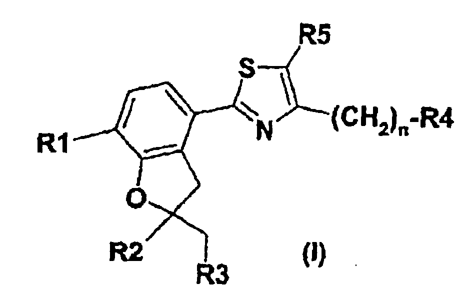 dans laquelle
R1 représente un reste alcoxy en C₁-C₄,
R2 et R3 représentent ensemble, avec les deux atomes de carbone auxquels ils sont liés, un cycle cyclopentane,
R4 représente un cycle phényle substitué par R41 ou un reste pyridine substituée par R44, où
R41 représente l'hydrogène ou un reste carboxyle, (alcoxy en C₁-C₄)carbonyle, carbamoyle, hydroxysulfonyle, sulfamoyle ou hydroxy, et
R44 représente l'hydrogène ou un reste carboxyle, (alcoxy en C₁-C₄)carbonyle, carbamoyle, hydroxysulfonyle, sulfamoyle ou hydroxy,
R5 représente l'hydrogène,
n est 0
et les sels de ces composés.

2. Composés de formule I selon la revendication 1, dans lesquels
R1 représente un reste méthoxy,
R2 et R3 représentent ensemble, avec les deux atomes de carbone auxquels ils sont liés, un cycle cyclopentane,
R4 représente un cycle phényle substitué par R41 ou un reste pyridine substituée par R44, où
R41 représente un reste carboxyle ou carbamoyle, et
R44 représente l'hydrogène ou un reste carboxyle ou (alcoxy en C₁-C₄)carbonyle,
R5 représente l'hydrogène,
n est 0
et les sels de ces composés.

3. Médicaments contenant un ou plusieurs composés selon la revendication 1 avec des agents auxiliaires et/ou des supports pharmaceutiques classiques.

4. Composés selon la revendication 1, à utiliser dans le traitement de maladies.

5. Utilisation de composés selon la revendication 1 pour la préparation de médicaments destinés au traitement de maladies des voies respiratoires.
